# EUROPEAN PATENT APPLICATION

(11) **EP 2 080 523 A1**
(43) Date of publication of application: **22.07.2009**
(21) Application number: 08000601.8
(22) Date of filing: 15.01.2008
(51) Int. Cl.: A61K 45/06, A61P 11/00

(54) **Compositions comprising an antimuscarinic and a long-acting beta-agonist**

(71) Applicant: CHIESI FARMACEUTICI S.p.A., I-43100 Parma (IT)
(72) Inventor: Villetti, Gino, 43100 Parma (IT); Razzetti, Roberta, 43100 Parma (IT)
(74) Representative: Minoja, Fabrizio

(57) **Abstract**

The invention relates to a composition comprising a combination of a salt of 3-[[[(3-fluorophenyl)[(3,4,5-trifluoro-phenyl)methyl]amino] carbonyl]-oxy]-1-[2-oxo-2-(2-thienyl)ethyl]-1-azoniabicyclo [2.2.2]octane, and a long-acting phenylalkylamino beta₂-agonist.

The invention also relates to pharmaceutical formulations and kit thereof, and to their use in the treatment of an inflammatory or obstructive airways disease.

## Description

### FIELD OF THE INVENTION

The invention relates to a composition comprising a combination of a salt of 3-[[[(3-fluorophenyl)[(3,4,5-trifluorophenyl)methyl]amino]carbonyl]-oxy]-1-[2-oxo-2-(2-thienyl)ethyl]-1-azoniabicyclo [2.2.2]octane, and a long-acting phenylalkylamino beta₂-agonist.

The invention also relates to pharmaceutical formulations and kit thereof, and to their use in the prevention and/or treatment of an inflammatory or obstructive airways disease.

### BACKGROUND TO THE INVENTION

Quaternary ammonium salts acting as muscarinic receptors antagonists are currently used in therapy to induce bronchodilation for the treatment of respiratory diseases, and in particular inflammatory or obstructive airway diseases such as asthma and chronic obstructive pulmonary disease (COPD).

For treating chronic diseases, it is often desirable to utilize antimuscarinic drugs with a long-lasting effect. This ensures that the concentration of the active substance necessary for achieving the therapeutic effect is present in the lungs for a long period of time, without the need for the active substance to be administered repeatedly and too frequently.

In particular, it would be desirable to utilize antimuscarinic drugs which are therapeutically efficacious upon administration by inhalation once a day.

In order to fulfill such a requirement, antimuscarinic drugs shall exhibit good selectivity for M3 muscarinic receptors, and slow dissociation from them.

Recently, it has been reported that tiotropium bromide, the first drug in a new generation of antimuscarinic drugs, exhibits a very slow dissociation from M3 receptors, behavior thought to account for its long lasting activity. However tiotropium bromide still retains a slow dissociation kinetics for the M2 muscarinic receptors. Since M2 receptors are a major population in the cardiac muscle, a therapy with said drug might be accompanied by undesired cardiac side effects.

The quaternary ammonium salt of 3-[[[(3-fluorophenyl)[(3,4,5-trifluoro phenyl)methyl]amino]carbonyl]oxy]-1-[2-oxo-2-(2-thienyl)ethyl]-1-azoniabicyclo-[2.2.2]octane (hereinafter referred to as active compound **1)** is a novel compound which has been disclosed in the co-pending Patent Application no. PCT/EP2007/057585. The preparation of the (R) enantiomer of compound **1** is detailed in the Example 1 below.

The active compound **1** has the following structure: 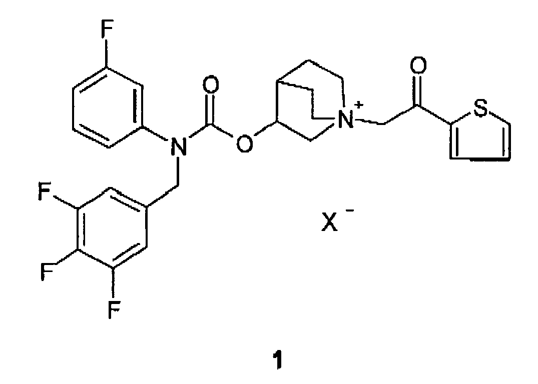 wherein X- is a pharmaceutically acceptable anion, preferably selected from the group consisting of chloride, bromide, iodide, sulfate, phosphate, methanesulfonate, nitrate, maleate, acetate, citrate, fumarate, tartrate, oxalate, succinate, benzoate and p-toluenesulfonate.

In particular the chloride salt of active compound **1** has been found to be equieffective to tiotropium bromide in terms of receptor potency and duration of action, but significantly short-acting on the M2 receptors.

Therefore a salt of active compound **1** may provide significant therapeutic benefit in the treatment of respiratory diseases such as asthma and COPD, when administered by inhalation.

In particular said active drug could provide antimuscarinic-based pharmaceutical compositions provided with a rapid onset of action and a long-lasting effect in such a way as it could be administered once a day with a great improvement of the compliance of patients.

Moreover said pharmaceutical compositions could turn out to be safer with respect to those of the prior art.

It has now being found that an unexpectedly beneficial therapeutic effect, particularly a synergistic effect, is observed in the treatment of inflammatory or obstructive diseases of the respiratory tract if compound **1** is used in combination with a long-acting beta₂-agonist.

The combination might also allow to use a lower dose of long-acting beta₂-agonists reducing the undesirable side effects which often occur, upon their administration. Undesirable side effects in this context are, in particular, the stimulant effects on the heart which are sometimes caused by beta₂-agonists, especially tachycardia, palpitations, angina-pectoris-like pain and arrhythmia.

### SUMMARY OF THE INVENTION

The invention provides a composition comprising a pharmaceutically acceptable salt of 3-[[[(3-fluorophonyl)[(3,4,5-trifluorophenyl)methyl]amino]-carbonyl]oxy]-1-[2-oxo-2-(2-thienyl)ethyl]-1-azoniabicyclo [2.2.2] octane (active compound **1)** in combination with a long-acting phenylalkylamino beta₂-agonist (active compound **2)** or a pharmaceutically acceptable salt thereof.

In a preferred embodiment, the composition comprises the active compound **1** in combination with formoterol or carmoterol as compound **2.**

In the compositions, the active compounds may be combined in a single preparation or contained in two separate formulations. Pharmaceutical compositions which comprise the active compound **1** and an active compound **2** in a single preparation are preferred.

Accordingly, the present invention relates to a pharmaceutical composition comprising an effective amount of the active compound **1** in combination with an effective amount of an active compound **2** and optionally at least one pharmaceutically acceptable carrier.

The invention also provides a device comprising said pharmaceutical formulation.

The invention further provides a kit comprising the active compound **1** and an active compound **2** in separate unit dosage forms, said forms being suitable for administration of the active compounds **1** and **2** in effective amounts.

The invention also relates to the use of the active compound **1** in combination with an active compound **2** as a medicament.

Moreover the invention relates to the use of the active compound **1** in combination with an active compound **2** in the preparation of a pharmaceutical composition or a kit for the prophylaxis or treatment, by separate, simultaneous or sequential administration of the active compound **1** and an active compound **2,** of an inflammatory or obstructive airways disease, such as asthma or chronic obstructive pulmonary disease (COPD).

Finally the invention relates to a method for the prevention and/or treatment of an inflammatory or obstructive airways disease treating respiratory disorders such as asthma or chronic obstructive pulmonary disease (COPD), said method comprising simultaneous or sequential administration of an effective amount of the active compound **1** in combination with a an active compound **2.**

### DEFINITIONS

"Long-acting beta₂-agonist" means a compound that is administered not more frequently than twice a day, preferably once a day.

The terms "active drug", "active ingredient", "active", "active compound" "active substance", and "therapeutic agent" are used as synonyms.

The terms "muscarinic receptor antagonists", antimuscarinic drugs" and "anticholinergic drugs" are used as synonyms.

"Full single therapeutically effective dose" means the quantity of active ingredient administered at one time by inhalation upon actuation of the inhaler.

Said full single dose may be delivered in one or more actuations, preferably one or two actuations (shots) of the inhaler.

"Actuation" means the release of the active ingredient from the device by a single activation (e.g. mechanical or breath).

As used herein, the term "substantially pure" means an active ingredient having an optical purity higher than 95% w/w, preferably higher than 98% w/w.

As used herein, the term "fixed combination" means a combination wherein the active substances are in a fixed amount and quantitative ratio.

As used herein, the term "synergistic" means that the activity of the two compounds is more than would be expected by summing their respective individual activities in a given assay.

### DETAILED DESCRIPTION OF THE INVENTION

The invention relates to a composition comprising the active compound **1** in combination with an active compound **2.**

In particular the invention is directed to a composition comprising a fixed combination of active compound **1** and an active compound **2.**

Within the scope of the present invention, any reference to the active compound **1** is to be regarded as a reference to a pharmaceutically acceptable salt of 3-[[[(3-fluorophenyl)[(3,4,5-trifluoro phenyl)methyl]amino] carbonyl]oxy]-1-[2-oxo-2-(2-thienyl)ethyl]-1-azoniabicyclo [2.2.2] octane, whose formula is reported below 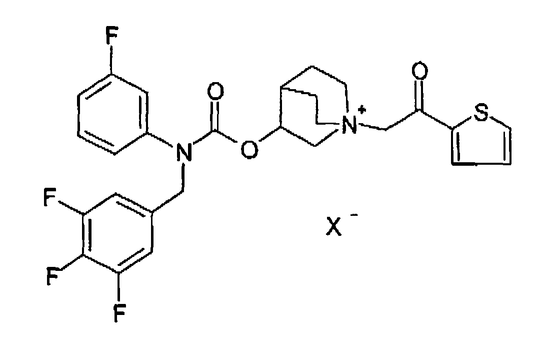 wherein X- is a pharmaceutically acceptable anion, preferably selected from the group consisting of chloride, bromide, iodide, sulfate, phosphate, methanesulfonate, nitrate, maleate, acetate, citrate, fumarate, tartrate, oxalate, succinate, benzoate and p-toluenesulfonate.

The active compound **1** is preferably used in the form of chloride salt.

It will be apparent that the active compound **1** displays an asymmetric carbon on the quinuclidine ring, and hence may be in the form of two optical stereoisomers, (3R)- and (3S)-stereoisomers or a racemic mixture thereof.

The active compound **1** is preferably in the form of the substantially pure (3R)-enantiomer.

The (3R)-enantiomer of active compound **1** in the form of chloride salt is hereinafter referred to as compound **1".**

The intended dose regimen of salts of active compound **1** is twice or once daily, preferably once daily, where the suitable daily dose is advantageously in the range of about 1 to about 160 µg, preferably between about 5 and about 80 µg expressed as compound **1** depending on the patient (age, weight and so on) and on the kind and the severity of the disease.

Within the scope of the present invention, any reference to the active compound **2** is to be regarded as a reference to a long-acting phenylalkylamino beta₂-agonist or a pharmaceutically acceptable salt thereof, also including the relevant enantiomers or mixtures thereof.

Examples of physiologically acceptable acid addition salts of the active compound **2** according to the invention are the pharmaceutically acceptable salts which are selected among the salts of hydrochloric acid, hydrobromic acid, sulfuric acid, phosphoric acid, methanesulfonic acid, acetic acid, fumaric acid, succinic acid, lactic acid, citric acid, tartaric acid or maleic acid.

The active compounds **2** may also be present according to the invention in the form of hydrates or solvates thereof.

The compositions according to the invention may comprise the compound **1** and the compound **2** in ratios ranging from 200:1 to 1: 500.

Advantageously, the compound **2** is selected from the group consisting of formoterol, hereinafter also indicated as compound **2a** and salmeterol, hereinafter also indicated as compound **2b,** or salts thereof. The salts of salmeterol and formoterol also include the relevant enantiomeric salts of (R)-salmeterol, (S)-salmeterol, (R,R)-formoterol, (S,S)-formoterol, (R,S)-formoterol, (S,R)-formoterol, and the mixtures thereof, while the enantiomeric salts of (R)-salmeterol and the racemic mixture (R,R)(S,S)-formoterol are of particular importance.

Formoterol is preferably used in the form fumarate salt, more preferably in the form of dihydrate fumarate salt, hereinafter also indicated as compound **2a",** while salmeterol is preferably used as xinafoate salt, hereinafter also indicated as compound **2b".**

The active compound **2** may also be a quinolinone derivative belonging to the formula **A:** 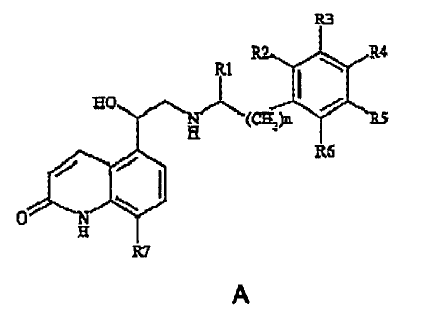 wherein
- R₁ is methyl and R₂ is hydrogen or R₁ and R₂ form a methylene bridge - (CH₂)ₙ- with n is 1 or 2, preferably 1,
- R₃, R₄, R₅ and R₆ are each independently hydrogen, hydroxy, a straight chain or branched C₁-C₄ alkyl, a straight chain or branched C₁-C₄ alkyl substituted with one or more halogen atoms and/or hydroxy groups, halogen, straight chain or branched C₁-C₄ alkoxy, and
- R₇ is hydrogen, hydroxy, straight chain or branched C₁-C₄ alkyl, straight chain or branched C₁-C₄ alkoxy.

Particularly preferred is the active compound wherein R₁ is methyl, R₄ is methoxy, R₂, R₃, R₅, R₆ are hydrogen, R₇ is hydroxy, and n=1, that is the 8-hydroxy-5-[1-hydroxy-2-[2-(4-methoxyphenyl)-1-methylethyl]amino]ethy]-2(1H)-quinolinone, hereinafter also indicated as compound **2c.**

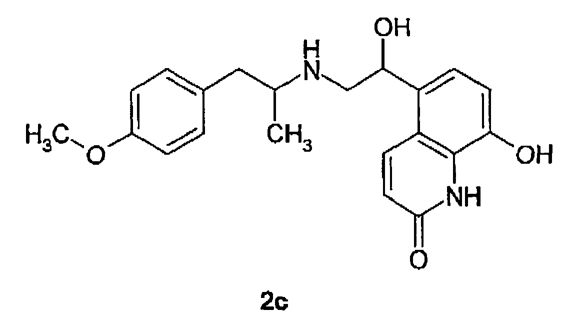

It will be apparent that said compound **2c** displays two asymmetric carbons at the position -CH(CH₃)- and at the position -CH(OH)-, respectively.

Therefore compound **2c** may exist in the form of four different stereoisomers, e.g (R,R)-, (R,S)-, (S,S)-, (S,R)- or mixtures thereof.

In a preferred embodiment the active compound **2c** is in the form of the optical pure (R,R)-enantiomer which has been also reported with the name of carmoterol or the experimental codes of TA 2005 and CHF 4226.

More preferably carmoterol is used as hydrochloride salt, hereinafter indicated as compound **2c".**

Another preferred quinoline derivative is the compound wherein R₁ and R₂ form a methylene bridge, R₃ and R₆ are H, R₄ and R₅ are ethyl, R₇ is OH, n = 1 which has also been reported with the name of indacaterol.

Indicaterol is hereinafter also indicated as compound **2d.**

Indicaterol is preferably used in the form of maleate salt, hereinafter also indicated as compound **2d".**

A further long-acting phenylalkylamino beta₂-agonist which might be advantageously used in combination with the active compound **1** is the compound milveterol (compound **2e)** or a pharmaceutically accepable salt thereof.

The salts of milveterol include the relevant enantiomeric salts (R,R), (S,S), (R,S), (S,R), and the mixtures thereof.

Preferably milveterol is used as hydrochloride salt (compound **2e"**).

Other long-acting phenylalkylamino beta₂-agonist which may be used in the combination of the invention are those known with the experimental codes PF-610355 and BI-1744-CL.

In a preferred embodiment, the composition comprises compound **1"** in combination with formoterol fumarate dihydrate.

In another preferred embodiment, the composition comprises compound **1"** in combination with carmoterol hydrochloride.

Within the scope of the present invention, the active compounds **1** and **2** may be administered simultaneously or sequentially, while it is preferable to administer active compounds **1** and **2** simultaneously.

In the medicament of the invention, the active compounds **1** and 2 may be combined in a single preparation or contained in two separate formulations.

Pharmaceutical compositions which comprise the active compounds **1** and 2 in a single preparation, and optionally a pharmaceutically acceptable carrier, are preferred.

The ratio by weight in which the active compounds **1** and **2** may be used in the pharmaceutical compositions according to the invention are variable.

The ratios may also vary on the basis of the different molecular weights of the various salt forms. The weight ratios specified below are based on compound **1",** the fumarate dihydrate salt of formoterol (compound **2a"**), the xinafoate salt of salmeterol (compound **2b"**), the hydrochloride salt of carmoterol (compound **2c"**), the maleate salt of indacaterol (compound **2d"**) and the hydrochloride salt of milveterol (compound **2e"**).

For instance, the pharmaceutical compositions according to the invention may comprise the compound **1"** and the compound **2a"** in ratios ranging from 1:24 to 40:1, preferably from 1:2 to 20:1, more preferably from 2:1 to 10:1.

In another embodiment, the compositions may comprise compound **1"** and the compound **2b"** in ratios ranging from 4:1 to 1:50, preferably from 1:1 to 1:20.

In another embodiment, the compositions may comprise compound **1"** and the compound **2c"** in ratios ranging from 80:1 to 1:8, preferably from 40:1 to 1:4, more preferably from 10:1 to 1:1.

In an further embodiment, the compositions may comprise the compound **1"** and the compound **2d"** in ratios ranging from 1:1 to 1:400, preferably from 1:10 to 2:100.

In an further embodiment, the compositions may comprise compound **1"** and the compound **2e"** in ratios ranging from 40:1 to 1:40.

The compositions of the invention may be administered one or more times a day, preferably once a day, and the single dose of active compounds **1** and **2** may vary depending on the disease and the conditions (weight, sex, age) of the patient.

The compositions according to the invention are normally administered so that the delivered single dose of active compound **1** is comprised between 2 µg and 80 µg, and that of active compound **2** is comprised between 0.5 µg and 400 µg.

Advantageously, when the compound **2** is formoterol, the compositions may deliver a single dose of compound **1",** comprised between 2 µg and 80 µg, preferably between 2 µg and 40 µg, and a single dose of formoterol, calculated as compound **2a",** comprised between 3 µg and 24 µg.

In one embodiment the single dose of compound **2a"** may be comprised between 3 µg and 6 µg.

In another embodiment the single dose of compound **2a"** may be comprised between 6 µg and 12 µg. In certain further embodiments, the single dose of compound **2a"** may be comprised between 12 µg and 18 µg or between 18 µg and 24 µg.

According to one embodiment the compositions of the invention may comprise a quantity of compound **1"** and compound **2a"** such that a single dose comprised between 5 µg and 40 µg of compound **1"** and of 3 µg of compound **2a"** are delivered.

In another embodiment a single dose comprised between 5 µg and 40 µg of compound **1"** and of 4 µg of **2a"** are delivered.

In a further embodiment, a single dose comprised between 5 µg and 40 µg of compound **1"** and of 6 µg of **2a"** are delivered.

In certain embodiments a single dose comprised between 5 µg and 40 µg of compound **1"** and of 6 µg, or 8 µg, or 10 µg, or 12 µg, or 15 µg, or 18 µg or 24 µg of **2a"** are delivered.

Advantageously, when the compound 2 is salmeterol, the compositions may deliver a single dose of compound **1",** comprised between 2 µg and 80 µg, preferably between 5 µg and 40 µg and a single dose of salmeterol, calculated as compound **2b",** comprised between 12 µg and 50 µg. In one embodiment the single dose of compound **2b"** may be comprised between 12 µg and 25 µg. In another embodiment the single dose of compound **2b"** may be comprised between 25 µg and 50 µg.

In one embodiment the compositions according to the invention may contain a quantity of compound **1"** and compound **2b"** such that a single dose comprised between 5 µg and 40 µg of compound **1"** and of 12 µg of compound **2b"** are delivered.

In another embodiment a single dose comprised between 5 µg and 40 µg of compound **1"** and of 25 µg of **2b"** are delivered.

In a further embodiment, a single dose comprised between 5 µg and 40 µg of compound **1"** and of 50 µg of **2b"** are delivered.

Advantageously, when the compound **2** is carmoterol the compositions may deliver a single dose of compound **1",** comprised between 2 µg and 80 µg, preferably between 5 µg and 40 µg, and a single dose of carmoterol, calculated as compound **2c",** comprised between 0.5 µg and 8 µg.

In one embodiment the single dose of compound **2c"** is comprised between 0.5 µg and 2 µg. In another embodiment the single dose of compound **2c"** may be comprised between 2 µg and 4 µg. In a further embodiment, the single dose of compound **2c"** may be comprised between 4 µg and 8 µg.

In certain embodiments single doses of 1 µg or 2 µg or 4 µg of compound **2c"** might be delivered.

For example, in one embodiment the composition according to the invention may comprise a quantity of compound **1"** and compound **2c"** such that a single dose of compound **1"** comprised between 10 µg and 40 µg and a single dose of **2c"** comprised between 1 and 4 µg are delivered.

In a particular embodiment a single dose of 10 µg of compound **1"** and of 2 µg of **2c"** are delivered. In a further particular embodiment, a single dose of 10 µg or 20 µg of compound **1"** and of 4 µg of **2c"** are delivered.

Advantageously, when the compound **2** is indacaterol, the compositions may deliver a single dose of compound **1",** comprised between 2 µg and 80 µg, preferably between 5 µg and 40 µg, and a single dose of indacaterol, calculated as compound **2d",** comprised between 25 µg and 200 µg.

In one embodiment the single dose of compound **2d"** is comprised between 25 µg and 50 µg. In another embodiment the single dose of compound **2d"** is comprised between 50 µg and 100 µg. In a further embodiment, the single dose of compound **2d"** is comprised between 100 µg and 200 µg.

In one embodiment the compositions according to the invention may comprise a quantity of compound **1"** and compound **2d"** such that a single dose comprised between 5 µg and 40 µg of compound **1"** and of 25 µg of **2d"** are delivered.

In another embodiment a single dose comprised between 5 µg and 40 µg of compound **1"** and of 50 µg of **2d"** are delivered. In a further embodiment, a single dose comprised between 5 µg and 40 µg of compound **1"** and of 100 µg or 200 µg of **2d"** are delivered.

Advantageously, when the compound **2** is milveterol, the compositions may deliver a single dose of compound **1"** comprised between 2 µg and 80 µg, preferably between 5 µg and 40 µg, and a single dose of milveterol, calculated as compound **2e",** comprised between 1 µg and 20 µg.

The quantities of active substance in the pharmaceutical compositions according to the invention which are administered per single dose can be calculated analogously if instead of the mentioned salts of the compounds **2,** other salts are used.

The pharmaceutical compositions according to the invention may optionally comprise a further active ingredient useful for the prevention and/or treatment of an inflammatory or obstructive airway disease.

According to a particular embodiment, the compositions of the invention may further comprise a corticosteroid, preferably selected from the group consisting of beclomethasone dipropionate (BDP), budesonide and epimers thereof, flunisolide, fluticasone propionate, mometasone furoate, ciclesonide, triamcinolone acetonide, rofleponide palmitate. More preferably the corticosteroid is beclomethasone dipropionate or budesonide.

The compositions of the invention may be preferably administered by inhalation. Inhalable preparations include inhalable powders, propellant-containing metering aerosols or propellant-free inhalable formulations.

For administration as a dry powder, single- or multi-dose inhaler device known from the prior art may be utilised, wherein the powder can be filled in gelatine, plastic or other capsules, cartridges or blister packs or in a reservoir. Said devices are known as dry powder inhalers (DPIs).

A diluent or carrier, generally non-toxic and chemically inert to the medicaments, e.g. lactose or any other additive suitable for improving the respirable fraction can be added to the powdered medicament.

Inhalation aerosols containing propellant gas such as hydrofluoroalkanes may contain the active ingredients of the combination of the invention either in solution or in dispersed form. Said propellant-driven formulations may also contain other ingredients such as co-solvents, stabilizers such as inorganic acids and optionally other excipients.

They are usually administered by inhaler devices known as metered dose inhalers (MDIs).

The propellant-free inhalable formulations comprising the combination of the invention may be in form of solutions or suspensions in an aqueous, alcoholic or hydroalcoholic medium and they may be delivered by jet or ultrasonic nebulisers known from the prior art or by inhaler devices known as soft-mist nebulisers (for example the nebuliser sold under the registered name of Respimat™).

Treatment of inflammatory or obstructive airways diseases in accordance with the invention may be symptomatic or prophylactic. Inflammatory or obstructive airways diseases to which the claimed combinations are applicable include asthma of whatever type or genesis including both intrinsic (non-allergic) asthma and extrinsic (allergic) asthma, mild asthma, moderate asthma, severe asthma, bronchitic asthma, exercise-induced asthma, occupational asthma and asthma induced following bacterial infection. Treatment of asthma is also to be understood as embracing treatment of subjects, e.g. of less than 4 or 5 years of age, exhibiting wheezing symptoms and diagnosed or diagnosable as "wheezy infants", an established patient category of major medical concern. Prophylactic efficacy in the treatment of asthma will be evidenced by reduced frequency or severity of symptomatic attack, e.g. of acute asthmatic or bronchoconstrictor attack, improvement in lung function or improved airways hyperreactivity. It may further be evidenced by reduced requirement for other, symptomatic therapy. Prophylactic benefit in asthma may in particular be apparent in subjects prone to "morning dipping". "Morning dipping" is a recognised asthmatic syndrome, common to a substantial percentage of asthmatics and characterised by asthma attack, e. g., between the hours of about 4 to 6 a.m., i. e. at a time normally substantially distant form any previously administered symptomatic asthma therapy.

Other inflammatory or obstructive airways diseases and conditions to which the present invention is applicable include acute lung injury (ALI), adult respiratory distress syndrome (ARDS), chronic obstructive pulmonary, airways or lung disease (COPD, COAD or COLD) including chronic bronchitis and emphysema, bronchiolitis, bronchiectasis and exacerbation of airways hyperreactivity consequent to other drug therapy, in particular other inhaled drug therapy.

Further inflammatory or obstructive airways diseases to which the present invention is applicable include pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tobacosis and byssinosis.

In particular, the combination of the invention is useful for the prevention and/or treatment of the chronic obstructive pulmonary disease (COPD), including chronic bronchitis and emphysema, bronchiolitis, and bronchiectasis, as in COPD patients, the antimuscarinic drugs relieve the airways constriction due to the vagal cholinergic tone.

The invention further provides a pharmaceutical kit comprising the active compound **1** and an active compound **2** in separate unit dosage forms, said forms being suitable for separate, simultaneous or sequential administration of the active compounds **1** and **2** in effective amounts.

Such a kit suitably further comprises one or more inhalation devices for administration of active compounds **1** and **2.**

For example, the kit may comprise one or more dry powder inhalation devices adapted to deliver dry powder from a capsule, together with capsules containing a dry powder comprising a dosage unit of the active compound **1** and capsules containing a dry powder comprising a dosage unit of an active compound **2.**

In another example, the kit may comprise a multidose dry powder inhalation device containing in the reservoir thereof a dry powder comprising the active compound **1** and a multidose dry powder inhalation device containing in the reservoir thereof a dry powder comprising an active compound **2**.

In a further example, the kit may comprise a metered dose inhaler containing an aerosol comprising the active compound **1** in a propellant, and a metered dose inhaler containing an aerosol comprising an active compound 2.

The invention is better illustrated by the following examples.

### EXAMPLES

### Example 1 - Preparation of (3R)-3-[(3-Fluorophenyl)-(3,4,5-trifluorobenzyl)carbamoyloxy]-1-(2-oxo-2-thiophen-2-yl-ethyl)-1-azoniabicyclo[2.2.2]octane chloride (compound 1')

A solution of (3R)-(3-fluorophenyl)-(3,4,5-trifluorobenzyl)carbamic acid 1-aza-bicyclo[2.2.2]oct-3-yl ester hydrochloride (prepared as described in WO03/053966 as Intermediate 15) (1 g, 2.248 mmol) in water (5 ml) was added with an excess of sodium carbonate (1 g) in water (4 ml) and extracted with ethyl acetate (2x10 ml). The organic layer was dried over anhydrous sodium sulfate and evaporated under vacuum.

The resulting free base (750 mg, 1.836 mmol) was dissolved in acetonitrile (4 ml) and added dropwise with a solution of 2-chloro-1-thiophen-2-yl-ethanone (750 mg, 3.657 mmol) in chloroform (6 ml). The resulting solution was refluxed for 12 hours. The solvent was evaporated and the residue dissolved in acetonitrile (7.5 ml). Crystallisation occurred after some minutes of stirring. The solid was filtered, washed with acetonitrile (1 ml) and dried under vacuum at 45°C. 760 mg of the title compound was obtained as white solid.

Mother liquors were evaporated to dryness and purified by column chromatography (SiO₂, eluent: dichloromethane/methanol =95/5 v/v to 80/20 v/v) to give further 120 mg of the title compound.

### Example 2 - Formulations for metered dose inhalers

Different formulations for metered dose inhalers are prepared with the following compositions:

| *Formulation* | *Amounts* | | |
|---|---|---|---|
| | Per unit | | Single dose |
| **(a)0** | mg | % (w/w) | µg |
| Compound **1"** | 1.60 | 0.017 | 10 |
| Formoterol fumarate*2H₂O | 0.96 | 0.01 | 6 |
| Ethanol | 1113.6 | 12 | |
| Hydrochloric acid 1 M | 0.014 | 0.00015 | |
| HFA134a | q.s. to 9280 | | |

| **(b)** | | | |
|---|---|---|---|
| Compound **1"** | 0.31 | 0.0028 | 2 |
| Carmeterol hydrochloride | 0.31 | 0.0028 | 2 |
| Ethanol | 1650.0 | 15 | - |
| Phosphoric acid 15.2 M | 0.3 | 0.0027 | - |
| HFA 134a | q.s. to 11,000 | | |

| **(c)** | | | |
|---|---|---|---|
| Compound **1"** | 1.54 | 0.016 | 10 |
| Carmeterol hydrochloride | 0.70 | 0.006 | 4 |
| Ethanol | 1650.0 | 15 | |
| Phosphoric acid 15.2 M | 0.3 | 0.0027 | |
| HFA 134a | q.s. to 11,000 | 84.981 | |

| **(d)** | | | |
|---|---|---|---|
| Compound **1"** | 6.17 | 0.064 | 40 |
| Carmeterol hydrochloride | 0.70 | 0.006 | 4 |
| Ethanol | 1650.0 | 15 | |
| Phosphoric acid 15.2 M | 0.3 | 0.0027 | |
| HFA 134a q.s. to 9.72 ml | q.s. to 11,000 | 84.933 | - |

### Example 3 - Powder formulations for a dry powder inhalers

Different formulations for dry powder inhalers are prepared with the following compositions:

| *Formulation* | *Amounts* | | |
|---|---|---|---|
| | For shot of the inhaler | | Single dose |
| **(a)** | mg | % (w/w) | µg |
| Compound **1"** | 0.01 | 0.1 | 10 |
| Formoterol fumarate*2H₂O compound 2 | 0.006 | 0.06 | 6 |
| Alpha-lactose monohydrate | 9.959 | 99.59 | |
| Magnesium stearate | 0.025 | 0.25 | |
| Total weight | 10 | 100 | |

| **(b)** | | | |
|---|---|---|---|
| Compound **1"** | 0.01 | 0.1 | 10 |
| Carmoterol hydrochloride compound 2 | 0.004 | 0.04 | 4 |
| Alpha-lactose monohydrate | 9.961 | 99.61 | |
| Magnesium stearate | 0.025 | 0.25 | |
| Total weight | 10 | | |

### Example 4 - Assessment of the bronchodilation activity of compound 1"

Airway reactivity is measured using barometric plethysmography (Buxco, USA). Male guinea pigs (500-600 g) are individually placed in plexiglass chambers. After an acclimatisation period, animals are exposed to nebulised saline for 1 min to obtain airway baseline reading. This is followed by a 1 min challenge with nebulised acetylcholine (Ach) -2.5 mg/mL.

After 60 min, 5 min nebulisation of vehicle or the compound 1" in the range 2.5 -250 mM are applied and Ach challenge is then repeated after 2, 5, 24, 48 and 72 h. Recording of pressure fluctuations in the chambers are taken for 5 min after each nebulisation and analysed to calculate Enhanced Pause (Penh). Airway reactivity is expressed as percentage increase in Penh compared with Penh values from the nebulisation of vehicle.

Two hours after the end of nebulisation with compound **1"**, the Ach-induced increase in Penh is dose-dependently inhibited by the compound, with a maximal effect of 99.2 ± 0.5 at 250 mM.

As for the time-course of the effect, compound **1"** shows increasing duration of action with increasing dose.

After inhalation of 250 mM of compound **1"**, effect persists unchanged up to 48 h (83.0 ± 16.1 %), while at 72 h a residual activity of 34.8 ± 20.9% is present. Twenty-four hours after 25 and 50 mM compound **1"** inhalation, a significant bronchoprotective effect was observed (87.1 ± 8.7% and 63.1 ± 19.2%, respectively). At 50 mM, a significant inhibition persists up to 48 h (49.2 ± 23.2%). Inhalation of lower concentrations results in an effect that did not exceed the 5 h observation point.

The estimation of lung levels of compound **1"** achieved after nebulisation endowed with a submaximal bronchodilator activity at 2 h after treatment reveals that the its retained dose in the target organ is about 50 µg/kg. If an extrapolation of these results from rat to human is made, it can be predicted that in patients the therapeutically effective dose might be comprised between 1 and 80 µg, preferably between 5 and 40 µg, after administration by inhalation.

## Claims

1. A composition comprising:
(a) a pharmaceutically acceptable salt of formula **1** (active compound 1) 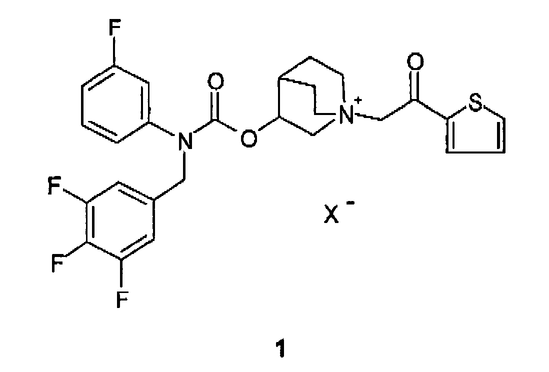 wherein:
X- is a pharmaceutically acceptable anion, and
(b) a long-acting phenylalkylamino beta₂-agonist (active compound 2),
or a pharmaceutically acceptable salt or a solvate or hydrate thereof, optionally in the form of the enantiomers or mixtures of the enantiomers.

2. The composition according to claim 1 wherein the anion is selected from the group consisting of chloride, bromide, iodide, sulfate, phosphate, methanesulfonate, nitrate, maleate, acetate, citrate, fumarate, tartrate, oxalate, succinate, benzoate and p-toluenesulfonate.

3. The composition according to claim 1 or 2 wherein the pharmaceutically acceptable salt of compound 2 is selected form the group consisting of the salts of hydrochloric acid, hydrobromic acid, sulfuric acid, phosphoric acid, methanesulfonic acid, acetic acid, fumaric acid, succinic acid, lactic acid, citric acid, tartaric acid and maleic acid.

4. The composition according to any one of claims 1 to 3, wherein the compound 1 and the compound 2 are present in a fixed combination.

5. The composition according to claim 4 wherein the compound **1** and the compound **2** are present in a ratio comprised between 200:1 and 1:500.

6. The composition according to any one of claims 1 to 5 wherein the compound **1** is the form of chloride salt.

7. The composition according to claim 6 wherein the compound **1** is administered at a full single therapeutically effective dose comprised between 1 µg and 80 µg.

8. The composition according to claim 7 wherein the single dose is comprised between 2 µg and 40 µg.

9. The composition according to any one of claims 1 to 8 wherein the single dose of compound **2** is comprised between 0.5 µg and 400 µg.

10. The composition according to any one of claims 4 to 9 wherein the compound **2** is formoterol (compound **2a**).

11. The composition according to claim 10 wherein formoterol is in form of fumarate dihydrate (compound **2a"**).

12. The composition according to 11 wherein the weight ratio between compound 1 and compound **2a"** ranges from 1:24 to 40:1.

13. The composition according to any one of claims 4 to 9 wherein the compound 2 is salmeterol (compound **2b**).

14. The composition according to claim 13 wherein salmeterol is in the form of xinafoate salt (compound **2b"**).

15. The composition according to claim 14 wherein the weight ratio between compound 1 and compound **2b"** ranges from 4:1 to 50:1.

16. The composition according to any one of claims 4 to 9 wherein the compound **2** is a compound of general formula **A** 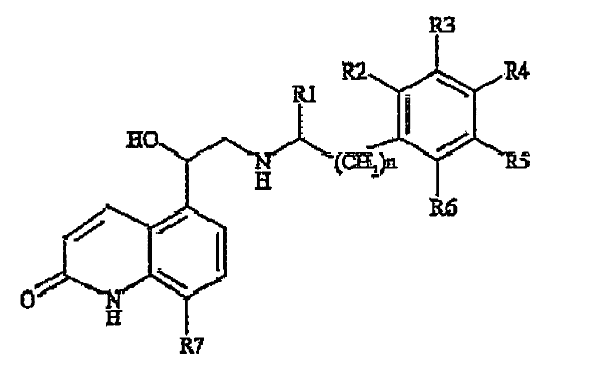 wherein
- R₁ is methyl and R₂ is hydrogen or R₁ and R₂ form a methylene bridge
- (CH₂)ₙ- with n is 1 or 2,
- R₃, R₄, R₅ and R₆ are each independently hydrogen, hydroxy, a straight chain or branched C₁-C₄ alkyl, a straight chain or branched C₁-C₄ alkyl substituted with one or more halogen atoms and/or hydroxy groups, halogen, straight chain or branched C₁-C₄ alkoxy, and
- R₇ is hydrogen, hydroxy, straight chain or branched C₁-C₄ alkyl, straight chain or branched C₁-C₄ alkoxy.

17. The composition according to claim 16 wherein the compound **2** is a compound of general formula **A** wherein R₁ is methyl, R₄ is methoxy, R₂, R₃, R₅, R₆ are hydrogen, R₇ is hydroxy, and n=1 (compound **2c**).

18. The composition according to claim 17 wherein the compound **2c** is in the form of hydrochloride salt (compound **2c"**).

19. The composition according to claim 18 wherein the weight ratio between compound **1** and compound **2c"** ranges from 80:1 to 1:8.

20. The composition according to claim 16 wherein the compound **2** is a compound of general formula **A** wherein R₁ and R₂ form a methylene bridge, R₃ and R₆ are H, R₄ and R₅ are ethyl, R₇ is OH, n = 1 (compound **2d**).

21. The composition according to claim 20 wherein the compound 2d is in the form of maleate salt (compound **2d"**).

22. The composition according to claim 21 wherein the weight ratio between compound **1** and compound **2c"** ranges from 1:1 to 1:400.

23. The composition according to any one of claims 4 to 9 wherein the compound **2** is milveterol or a salt thereof (compound **2e**).

24. A pharmaceutical composition comprising in admixture in a single preparation:
(a) a pharmaceutically acceptable salt of formula **1** (active compound **1**) 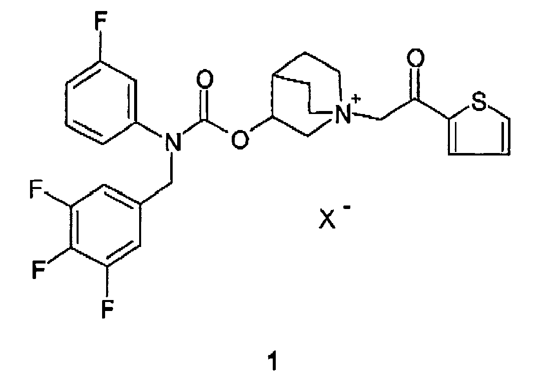 wherein:
X- is a pharmaceutically acceptable anion,
(b) a long-acting phenylalkylamino beta₂-agonist. **2** (active compound **2**),
or a pharmaceutically acceptable salt or a solvate or hydrate thereof, optionally in the form of the enantiomers, or mixtures of the enantiomers, and a pharmaceutically acceptable carrier.

25. The pharmaceutical composition according to claim 24 wherein the anion is selected from the group consisting of chloride, bromide, iodide, sulfate, phosphate, methanesulfonate, nitrate, maleate, acetate, citrate, fumarate, tartrate, oxalate, succinate, benzoate and p-toluenesulfonate.

26. The pharmaceutical composition according to claim 24 or 25 further comprising a corticosteroid.

27. The pharmaceutical composition according to claim 26 wherein the corticosteroid is selected form the group consisting of beclomethasone dipropionate (BDP), budesonide and epimers thereof, flunisolide, fluticasone propionate, mometasone furoate, ciclesonide, triamcinolone acetonide, rofleponide palmitate.

28. The pharmaceutical composition according to any one of claims 24 to 27 wherein the pharmaceutical composition is an inhalable aerosol comprising a propellant.

29. The pharmaceutical composition according to any one of claims 24 to 27 wherein the pharmaceutical composition is an inhalable powder.

30. The pharmaceutical composition according to any one of claims 24 to 27 wherein the pharmaceutical composition is an inhalable propellant-free solution or suspension.

31. A device comprising a pharmaceutical composition according to any one of claims 24 to 29.

32. A kit comprising:
a) a therapeutically effective amount of a pharmaceutically acceptable salt of formula **1** (active compound **1**) and, optionally, a pharmaceutically acceptable carrier in a first unit dosage form; 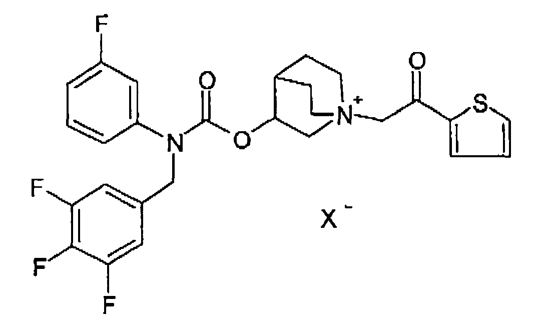 wherein:
X- is an anion selected from the group consisting of chloride, bromide, iodide, sulfate, phosphate, methanesulfonate, nitrate, maleate, acetate, citrate, fumarate, tartrate, oxalate, succinate, benzoate and p-toluenesulfonate, and
b) a therapeutically effective amount of a long-acting phenylalkylamino beta₂-agonist. **2** (active compound **2**) or a pharmaceutically acceptable salt thereof, and optionally a pharmaceutically acceptable carrier in a second unit dosage form;
for separate simultaneous or sequential administration in the prevention and/or treatment of an obstructive airways disease.

33. The kit according to claim **32** further comprising one or more inhaler devices.

34. Use of active compound **1** in combination with an active compound **2** as a medicament.

35. Use of active compound **1** in combination with an active compound **2** in the preparation of a pharmaceutical composition or a kit for the prophylaxis or treatment, by simultaneous or sequential administration of active compound **1** and an active compound **2**, of an inflammatory or obstructive airways disease.

36. The use according to claim 35 wherein the disease is asthma.

37. The use according to claim 35 wherein the disease is chronic obstructive pulmonary disease (COPD).

38. The use according to any one of claims 35 to 37 wherein the full single therapeutically effective dose of compound **1** is comprised between 1 µg and 80 µg.

39. The use according to claim 38 wherein the dose is comprised between 5 µg and 40 µg.
